# EUROPEAN PATENT APPLICATION

(11) **EP 4 445 995 A1**
(43) Date of publication of application: **16.10.2024**
(21) Application number: 23382338.4
(22) Date of filing: 12.04.2023
(51) Int. Cl.: B01F 23/235, B01F 27/118, B01F 33/452, B01F 35/10, B01F 35/71, B01F 35/75, B01F 35/221, A61J 1/14, B01F 101/22

(54) **METHODS AND SYSTEMS FOR PREPARING SCLEROSANT FOAMS**

(71) Applicant: Vascular Barcelona Devices, S.L., 08006 Barcelona (ES)
(72) Inventor: ROCHE REBOLLO, Enrique, 08034 Barcelona (ES); LLUSÀ MELÉNDEZ, Guiu, 08018 Barcelona (ES); GREGO MAYOR, Federico, 08021 Barcelona (ES)
(74) Representative: Bardehle Pagenberg S.L.

(57) **Abstract**

Methods for preparing a foamed sclerosant composition are provided. The methods may comprise providing a container 10 with a foaming space being defined in an interior of the container body, and a mixing component 30 comprising a magnetic part. The method may further comprise injecting a liquid sclerosant composition into the foaming space of the container, and providing a rotating magnetic field in order to rotate the mixing component. Providing the rotating magnetic field comprises providing a rotating magnetic field at a first speed, maintaining the rotating magnetic field at the first speed for a first holding time, providing a rotating magnetic field at a second speed, the second speed being higher at a first speed, and maintaining the rotating magnetic field at the second speed for a second holding time. Also magnetic stirrers specifically configured to carry out such methods are provided.

## Description

The present disclosure is related to the technical field of vascular medicine, and more particularly to the field of treatments for varicose veins and other vascular problems such as e.g. spider veins and/or haemorrhoids.

Specifically, the present disclosure relates to devices, systems, products and methods for obtaining a foam, which can be used for the treatment of the affected veins. The present disclosure further relates to the composition of the foam itself obtained by any of the procedures described throughout the present disclosure, as well as to the use of the devices, systems and products in order to treat varicose veins and other vascular problems such as e.g. spider veins and/or haemorrhoids. Furthermore, the present disclosure also relates to methods for the manufacture of such products to treat varicose veins and other vascular problems.

### BACKGROUND ART

Varicose veins occur when the venous valves (which prevent the backflow of blood) do not work properly. As a result, the vein walls are weakened, and they can become deformed and dilated. Due to the fact that the valves do not work properly, the blood may recirculate and short-circuits may be created. Subsequently, the veins may become progressively dilated. As a result, the varicose veins can become more visible, and can be full of bends, and become more voluminous. The evolution of this pathology may lead to consequences beyond mere cosmetic ones, such as discoloration of the skin, pain, and swelling of the extremities due to the effect of venous hypertension.

According to the Spanish Society of Angiology and Vascular Surgery (SEACV) in Spain, varicose veins affect 30 to 33 % of the adult population in industrialized countries.

The veins most commonly affected are those located in the legs, although varicose veins may occur interiorly as well: e.g. varicose veins in the esophagus, around organs located in the pelvis (pelvic and ovarian varicose veins) or at or near the most distal part of the digestive tube, near the anus (haemorrhoids).

Nowadays, there are many different treatments and/or strategies to mitigate or eliminate these problems.

A common technique to treat varicose veins is sclerotherapy. This technique is less aggressive and less painful than other techniques such as endovenous techniques or radiofrequency therapies. Further, sclerotherapy needs no anesthesia.

Sclerotherapy involves the injection of a liquid (or foam when shaken) with the ability to irritate the vascular endothelium, which is a thin layer or lining inside the vein that is in contact with the bloodstream. The products internationally approved for this use are lauromacrogol (also known named as polidocanol and commercially available as etoxiesclerol^{®}), and sodium tetradecyl sulfate.

The advantage of using such a product as a foam is based on the enhancement of its effect due to the larger contact surface with the endothelial wall. The larger contact surface offers the possibility of dose reduction. Also, the visibility of the drug as a foam using ultrasounds through the ultrasound scanner is improved (Schadeck M, Allaert FA. Duplex scanning in the mechanism of the sclerotherapy: Importance of the spasm. Phlebology 1995; Suppl1: 574-576).

The effect produced by the foam on the endothelium involves the injury of the cell layer of the vein, whereby thrombosis of its content is produced. Later, this vein suffers a fibrosis process (retraction and disposal) and the vein eventually may disappear after several months. This process can be faster or slower depending on the size of the vein or the potency of the varicose agent. Therefore, it is sometimes necessary to apply several sessions on the vein.

In recent years, this procedure has been developed a lot further and there has been a growing interest from the scientific community to obtain methods suitable for the manufacture of such foams in a safe and convenient way.

Although there are several methods to eliminate or remove varicose veins, the least aggressive and least disabling treatment so far, and the one which can be used for a wide variety of pathologies is the ultrasound-guided Foam Sclerotherapy using polidocanol or other sclerosing agents. Sclerotherapy is the least invasive treatment of varicose veins known to date as it can be performed in a physician's office and in a completely ambulatory way. Therefore, the present disclosure is focused on the use of this technique.

In 1995, Dr. Juan Cabrera presented the results of the application of a foam that he had developed with his son, the pharmacist Juan Cabrera (Cabrera J. et al. "Treatment of Varicose Long Saphenous Veins of Sclerosants in Microfoam Form With: Long-term Outcomes". Phlebology (2000) 15; 19-23). This foam was characterized by its density and high solubility thanks to the use of a mixture of physiological gases.

Furthermore, he managed to achieve a type of foam with a very small and uniform bubble size, thanks to his method of using a mixer. By using a mixture of physiological gases, the foam had greater security and stability. This foam was called "microfoam" due to the small bubble size, uniformity and stability.

Shortly after, the maximum popularization of the sclerosing foam came from Lorenzo Tessari (Tessari L., Cavezzi A., Frullini A., Preliminary experience with a new sclerosing foam in the treatment of varicose veins. Dermatol Surg 2001 Jan; 27 (1): 58-60) who published his experience using a foam easily manufactured through a procedure called "The Tessari Method". The method consists of agitating a sclerosing liquid using two syringes connected via a three-way tap. By means of successive alternately movements with each of the syringes connected to the gas/liquid mixture, a mix of foam, located at the inner part of the syringe, was achieved. However, this manufacturing technique, in spite of being the most widespread is not the most effective, since a relatively unstable and heterogeneous foam is obtained.

In recent years numerous articles and papers have been published about safety profiles, side effects, and potential complications arising from the use of these products. Thus, it appears demonstrated that the best foam used is the one whose gas is a mixture of O₂/CO₂ at different concentrations. With this arrangement, the solubility and diffusion in the blood are very high as opposed to atmospheric gas foams. Additionally, the foam stability is linked to the size of the bubble. Also, it has been found that the foam is more stable when the bubble diameter is more homogeneous.

As has already been mentioned, although there are a lot of manufacturing systems of sclerosing foam, the most common foam (and the one which is normally used around the world) is the foam obtained with the Tessari method. However, this method has many problems of standardization and homogenization. This system consists of mixing air flow with the selected liquid, either polidocanol or sodium tetradecyl sulfate (commercially known as sotradecol ^{®}). This foam can have medium size bubbles, but of irregular size and the foam becomes unstable after a few seconds of its formation. Additionally, the use of atmospheric gas as a vehicle for the sclerosing foam limits the foam administrable per session.

US2017144115 discloses a container for the production of a foamed sclerosant composition, kits and systems including such a container, and methods for preparing a foamed sclerosant composition using such containers. In an aspect, the container comprises a container body and a mixing element disposed in the container body, such that a foaming space is defined in an interior of the container body between the sidewalls and the mixing element. The mixing element may be configured to be operatively coupled with a rotating actuator without the actuator reaching the foaming space. In particular, the mixing element may have a magnetic core, such that the mixing element can be set in motion when placed on a magnetic stirrer.

WO2017085209 relates to a container for the production of a foamed sclerosant composition, to kits and systems including such a container, to methods for preparing a foamed sclerosant composition using such containers, and to foamed sclerosant compositions obtainable by such methods. In an aspect, the container comprises a sealed sterile container body having one or more sidewalls extending between a top and a bottom of the container body and defining a foaming space. The container further comprises a mixing element disposed in the container body. The container contains a previously introduced gas and liquid sclerosant composition. The mixing element is configured to be operatively coupled with a rotatable actuator without the actuator reaching the foaming space. A medical professional may select the appropriate quantity and concentration for every treatment.

WO2020038928 also relates to a container for the production of a foamed sclerosant composition, to kits and systems including such a container, to methods for preparing a foamed sclerosant composition using such containers, and to foamed sclerosant compositions obtainable by such methods. In an aspect, the container comprises a pressure equalizer for equalizing pressure inside and outside the foaming space.

Pending European patent application EP23382040.6 filed on January 18th, 2023 describes a container for the production of a foamed sclerosant composition which is pre-filled with a mixture of physiological gases.

These documents disclose methods and devices that facilitate quick production of good quality sclerosant foam with relatively simple tools and in a highly sterile manner.

However, systems and methods for providing sclerosant foam can still be further improved or adjusted with respect to the characteristics of the foam obtained.

In particular, the present disclosure focuses on the ratio of liquid to gas in the obtained foam. Throughout the present disclosure, the ratio of liquid to gas may be understood as the ratio between the volume of liquid and the volume of gas in a sclerosant foam. A ratio of 1:5 herein means that the volume of gas will be five times more than the volume of liquid. For example, if 12 ml sclerosant foam is obtained, 2 ml will be liquid and 10 ml will be gas. The ratio may be written as 1:5 as hereinbefore. Alternatively, the ratio may be written as 1/5.

The ratio of liquid to gas in the sclerosant foam affects the stability of the sclerosant foam, as well as the treatment. For example, a liquid to gas ratio of 1:2 or 1:3 is relatively unstable and the foam therefore doesn't last a long time. Although there is no general consensus on what the optimum ratio is for a specific treatment, it is generally accepted that higher ratios (i.e. more gas) of 1:5, 1:6 or 1:7 are more stable and thereby can increase the aforementioned irritation of the vascular endothelium.

However, apart from the systems and methods described in the herein cited patent applications, there are no systems available that can consistently, and for different sclerosant liquids, provide foams with a ratio of 1:5 or higher. The different sclerosant liquids behave differently as well: lauromacrogol (polidocanol or etoxiesclerol^{®}) has nonpolar covalent bonds, whereas sodium tetradecyl sulfate has polar bonds. Foams based on the use of sodium tetradecyl sulfate for this reason tend to be formed more quickly (when mixing liquid and gas) but also tend to be less stable than foams based on polidocanol.

### SUMMARY

In a first aspect, the present disclosure provides a method for preparing a foamed sclerosant composition. The method comprises providing a container having one or more sidewalls extending between a top and a bottom of the container, with a foaming space being defined in an interior of the container, and wherein the foaming space contains a mixing component configured for rotating on the bottom of the container body and comprising a magnetic part and a shape suitable for mixing. The method further comprises introducing a liquid sclerosant composition into the foaming space of the container and providing a rotating magnetic field in order to rotate the mixing component in the foaming space. Providing the rotating magnetic field comprises providing a rotating magnetic field at a first speed, and maintaining the rotating magnetic field at the first speed for a first holding time and subsequently providing a rotating magnetic field at a second speed, the second speed being higher at a first speed, and maintaining the rotating magnetic field at the second speed for a second holding time.

In accordance with this aspect, a method for preparing a foamed sclerosant composition is provided which has a staged preparation of foam, by changes of speed in rotation of the magnetic field. It has surprisingly been found that after an initial creation of foam (rotating at the first speed), and a certain stabilization of the foam (maintaining the first speed during a first holding time), a ratio of liquid:gas may be increased by an increase in speed of rotation. The staged increase of speed has been found to increase a liquid:gas ratio of a foam prepared with polidocanol from e.g. 1:5 to 1:7 or 1:8. Similar methods with Sodium tetradecyl sulfate (STS) may provide even higher liquid:gas ratios.

In some examples, the container may comprise a physiological gas or a mixture of physiological gases, and optionally may be a mixture of O₂ and CO₂. In some examples, the gas inside the container may comprise more than 95 % of physiological gases, and more specifically more than 98 % of physiological gases. In examples, the gas inside the container may be nearly 100 % physiological gas(es). Further, in some examples, the mixture of physiological gases may have a percentage of O₂ between 70% - 50 % and a percentage of CO₂ between 30 % - 50 %. The foam prepared with physiological gases may be suitable for the treatment of certain patients and/or veins which cannot be treated by foam prepared with (sterile) air.

In examples, the container may be provided in a package, and the gas inside the package may have a composition substantially similar to the gas composition of the container, particularly a physiological gas or mixture of physiological gases. In a specific example, the container may be filled with 65% O₂ and 35% CO₂. The same composition may be used in the packaging. Gas exchange can occur through the gas-permeable barrier of the container, but it will not modify the composition of the gas inside the container, and thus will not affect the composition of the foamed sclerosant composition.

In a further aspect, a method for preparing and extracting a foamed sclerosant composition is provided. The method comprises providing a container to produce a foamed sclerosant composition having a sterile container body, as hereinbefore explained. The method also comprises providing a syringe filled with a liquid sclerosant composition. Additionally, the method comprises mating the syringe with the coupling and injecting the liquid sclerosant composition into the foaming space, operating the actuator to rotate the mixing component configured for rotating on the bottom of the sterile container body and create a foamed sclerosant composition, and extracting the obtained foamed sclerosant composition.

In yet a further aspect, a magnetic stirrer is provided. The magnetic stirrer comprises a support for placement of a container with a foaming space being defined in an interior of the container body, and wherein the foaming space contains a mixing component. The magnetic stirrer further comprises an actuator for providing a magnetic field and a control system configured to control the actuator such that a rotating magnetic field is provided which is configured to rotate the mixing component in the foaming space. The rotating magnetic field comprises a rotating magnetic field at a first speed, during a first holding time and a rotating magnetic field at a second speed during a second holding time, wherein the second speed is higher at a first speed, and maintaining the rotating magnetic field at the second speed for a second holding time.

In some examples, the syringe used for the introduction of the liquid sclerosant composition remains mated with the female coupling member while the foam is being created. If the syringe remains coupled with the container, the chances of contaminating the foam are reduced. After the foam has been created, the same syringe may be used for aspiration or extraction of the foam. The gas-permeable barrier may also be useful to again equalize the pressures so that a good quality foam may be extracted.

In some examples, an actuator may be operated to create a foamed sclerosant composition, and the actuator may be operated at a lower speed of rotation while the foamed sclerosant composition is being extracted. In particular, higher speed of rotation may be used for rapid preparation of foam of good quality (e.g. small bubbles), and the rotation continues as the foam is being extracted. The continued rotation forces the created foam to the outer area of the foaming space, from where it can be aspirated.

In some examples, the foamed sclerosant composition obtainable by any of the methods, systems and products described herein may be for use in the treatment of varicose veins, spider veins or haemorrhoids.

### BRIEF DESCRIPTION OF THE DRAWINGS

Particular implementations of the present disclosure will be described in the following by way of non-limiting examples, with reference to the appended drawings, in which:
Figure 1 schematically illustrates an example of a container for producing a foamed sclerosant composition;
Figures 2A - 2F schematically illustrate an example of a mixing component that may be used in examples of the present disclosure;
Figure 3 schematically illustrates an example of a product for producing a foamed sclerosant composition;
Figures 4A - 4F schematically illustrates a sequence of steps in an example of a method for the production of a foamed sclerosant composition;
Figure 5 schematically illustrates an example of a method for preparing a foamed sclerosant composition;
Figure 6A schematically illustrates an example of a configuration of a magnetic stirrer; and
Figure 6B schematically illustrates an example of speed of a rotating magnetic field during a method for preparing a sclerosant foam.

### DETAILED DESCRIPTION

The expression "therapeutically effective amount" as used herein, refers to the amount of the foamed composition that, when administered, is sufficient to treat the diseases to which it is addressed. The specific dose which depends on both the volume and the drug concentration of the foamed sclerosant composition to obtain a therapeutic benefit may vary depending on the particular circumstances of each patient.

As previously mentioned, an aspect of the present disclosure relates to a foamed sclerosant composition obtainable by any of the methods herein described. The expression "obtainable by" is used herein for defining the foamed sclerosant composition by its preparation process. In particular, it refers to the foamed composition that can be obtained through the preparation process which comprises the previously commented steps. For the purposes of the present disclosure, the expressions "obtainable", "obtained" and similar equivalent expressions are used interchangeably and, in any case, the expression "obtainable" encompasses the expression "obtained". Throughout the present disclosure, the terms sclerosant and sclerosing are used interchangeably. Similarly, sclerosing foam, and foamed sclerosant composition are used interchangeably as well.

For the purposes of the present disclosure, the term foamed sclerosing composition refers to a composition of a foam capable of bringing about a sclerosing effect, i.e. a composition for use as a medicament for intravenous injection, which is capable of causing an injury to the vessel wall by endothelial vacuolation of the epithelial cell membrane (the layer in contact with the bloodstream). Thus, the foamed sclerosing composition irritates the inner surface of the vein just producing the formed thrombus formation by platelets and aggregates. Similarly, the term liquid sclerosing composition refers to a composition in liquid form including a sclerosing agent. The liquid sclerosing composition forms an ingredient to obtain the foamed sclerosing composition. The sclerosing compositions according to examples of the present disclosure comprise a sclerosing agent and also a suitable vehicle which can be injected without toxicity in the affected veins. In some examples, the liquid is selected from sterile water (particularly distilled water) and physiological saline. Examples of sclerosing agents that can be present in the sclerosing compositions of examples of the present disclosure include, without limitation, polidocanol, sodium tetradecyl sulfate, chromated glycerin, hypertonic saline, sodium morrhuate and sclerodex (hypertonic saline in combination with dextrose). In a particular example, the sclerosing composition used comprises polidocanol and water. In another embodiment, the sclerosing composition further comprises glycerin.

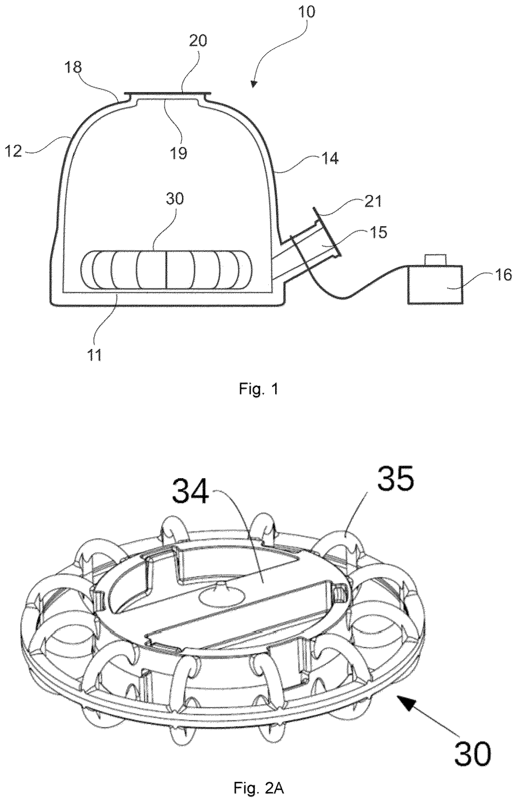

Figure 1 schematically illustrates an example of a container for producing a foamed sclerosant composition. A container 10 according to this example may include a dome shaped container body 12 with a bottom wall 11. The bottom wall 11 may be separately manufactured or may be manufactured integrally with the dome shaped container body 12. The dome shaped container body 12 may have a top 18 with an orifice 19. In this example, the orifice 19 is covered by a gas-permeable barrier 20. Even though the orifice 19 is herein depicted to be at the top of the container body 12, but in other examples, the orifice 19 may be located elsewhere in order to allow exchange of gases through the barrier 20, but avoiding contamination of particles, aerosols, liquids, other contaminants etc.

The dome shaped body 12 is formed by a curved side wall 14. Thus, a foaming space is defined in the interior of the container 10. The container 10, both dome shaped body 12 and bottom 11, may be made from a suitable polymer such as Polyethylene terephthalate (PET) or other suitable materials such as plastics generally accepted in the manufacture of medical devices. Other materials such as different types of glass with different material compositions, or materials with crystalline microstructure may also be used to manufacture the dome shaped body 12.

In some examples, the material of which the container is made is transparent to allow visual inspection of the formation of the foam. The dome shaped container body 12 and the bottom part 11 may be made by injection molding within the same mold or by separate molds. Other manufacturing processes could also be used.

If manufactured separately, the bottom 11 and the dome shaped body 12 may be joined by ultrasonic welding or other appropriate ways for joining plastic components. In some examples, a diameter of the container body (at the bottom) may be close to 4 cm. A height of the container body may be between 3,5 and 4 cm. It will be clear that teachings of the present disclosure also apply to containers with other dimensions.

A gas may be introduced into the foaming space during manufacture and assembly. The gas may be air but may also be a mixture of physiological gases, e.g. a mixture of carbon dioxide (CO₂) and oxygen (O₂).

More particularly, in specific examples, the mixture of physiological gases may comprise a percentage of O₂ between 70 % - 50 % and a percentage of CO₂ between 30 % - 50 %. Specifically, in an example, the mixture of physiological gases inside the container may be 65% of O₂ and 35% of CO₂ and with less than 0.8% of N₂. In practice, this corresponds to the mixture of physiological gases (O₂ and CO₂) constituting 99% or more of the gases contained in the container.

As provided, the foaming space also includes a mixing component 30 configured for rotating on the bottom 11 of the container 10. The container 10 with previously introduced gas and mixing component 30 for rotating may thus be provided to a medical professional.

The container 10 may be sterilized prior to providing it to a medical professional or it may be sterilized on site by a medical professional. Thus, in some examples the container 10 according to this example may be sterilized and wrapped. For example, plastic foil or wrapper may be provided around the container as delivered to a medical professional.

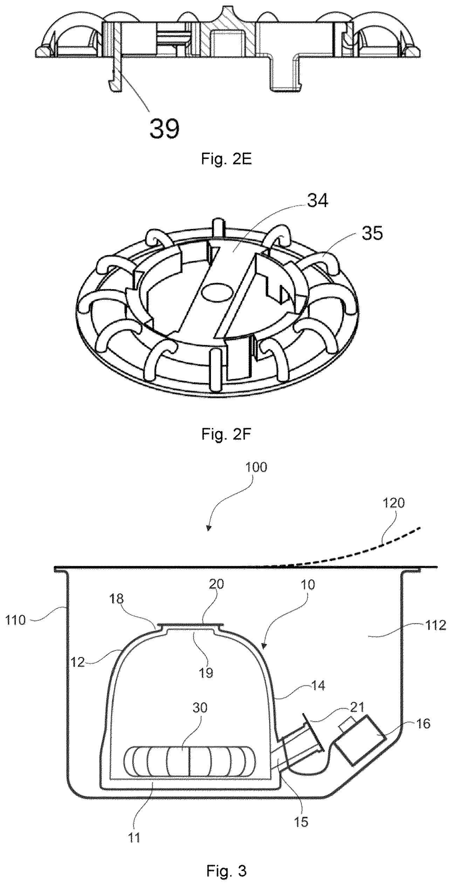

In other examples, the container 10 may be housed in a package, as will be discussed with respect to figure 3.

As shown in figure 1, the container 10 according to this example further includes a female coupling member 15 in the form of a socket with a stopper or lid 16. The female coupling member 15 may comprise external threads and the stopper 16 may comprise mating threads for easy attachment and detachment of the stopper. Alternative joints for female coupling member 15 and stopper 16 may however also be foreseen, e.g. a press or snap fit.

In examples, the female coupling member 15 may comprise a Luer taper, and particularly a Luer slip. The Luer taper may be sized such that it can mate with a tip of a syringe, in particular a hypodermic syringe. The tip of the syringe may be pressed into the Luer taper to provide a leak free fitting. In some examples, the syringe may be rotated to lock the syringe in the female coupling member 15 by means of a Luer lock.

In this example, the female coupling member 15 is integrally formed with the container 10 and in particular is provided on the sidewall 14, in proximity to the bottom 11. In some examples, the center of the female coupling member 15 is on the sidewall 14, at a height of 20 mm or less, i.e. up to a height of 20 mm from the bottom 11 of the container 10. Specifically, the center of the female coupling member 15 may be on the sidewall 14 up to 10 mm away from the bottom 11 of the container 10. However, other suitable positions for the female coupling member 15 may be envisaged. The stopper 16 may be made from polyethylene or other suitable materials.

Although not shown in this specific example, the female coupling member 15 or its connection to the foaming space may include a one-way valve, or any other element that facilitates passage from a liquid inside the syringe, and substantially impedes passage of gas from the inside to the outside.

In this example, the female coupling member 15 has an inclination with respect to a horizontal plane for easy insertion and extraction from the syringe.

An example of an assembly process of a container 10 is the following: after injection molding of the container 10, and before attachment of the bottom 11, the mixing component 30 is introduced into the foaming space of the container, and the housing is then closed. Sterile gas may be introduced through a suitable port, i.e. opening 19 and/or female coupling member 15 (e.g. air, and/or a mixture of physiological gases) forcing the evacuation of the gas previously present inside the container 10.

The gas-permeable barrier 20 of the container 10 may be a waterproof membrane, i.e. it may limit the ingress of liquid into the container 10. The waterproofing capacity of the membrane may be measured in terms of the height of the column of water that the membrane may hold before losing its permeability. In some examples, the waterproof membrane may have a waterproofing capacity of 1500 mm of column of water or more, and more specifically 5000 mm or more. Further, the gas-permeable barrier 20 may limit the penetration of aerosols and other suspension particles, which may contaminate the interior foaming space.

Additionally, the gas-permeable barrier 20 may have a relatively high capacity to transfer gas across the same.

In the present example, the gas-permeable barrier 20 comprises a polyethylene non-woven fabric, but other types of fabrics and materials may be used. For example, the barrier 20 may comprise polypropylene or other suitable materials.

The function of the gas-permeable barrier 20 is to balance pressure inside container 10 with pressure outside the container 10. Equilibration of pressures can avoid potential formation of undesirable large bubbles in the sclerosant foam.

As illustrated in figure 1, the female coupling member 15 may comprise a barrier 21, e.g. a gas-permeable barrier of the same of substantially similar characteristics to the gas-permeable barrier 20 covering the opening 19. Barriers with other mass transfer characteristics may also be used, e.g. a gas-impermeable barrier. The barrier 21 may protect the female coupling member 15 against the ingress of contaminants.

Barriers 20, 21 may be made of medical paper or may be made of Tyvek^{®} commercially available from DuPont. Tyvek^{®} is made of synthetic flashspun high-density polyethylene fibers. Tyvek^{®} is waterproof but allows water vapor to penetrate. In one example, Tyvek^{R} Allover 2FS/ACT2100^{™} may be used as material for the barrier. This product corresponds to Tyvek^{R} 2FS coated with a heat sealable coating and is a known packaging material for medical products. The air permeability measured with the aforementioned Bendsten mention is around 200 ml/min. The material is also suited for sterilization, e.g. with irradiation or ethylene oxide.

The barrier may be attached to the container to cover the opening(s). The barrier may be adhesively attached, or through ultrasound welding, or heat sealing or any other suitable method. In examples, the barrier(s) may be arranged at a substantially flat outer surface. For example, the dome-shaped walls may comprise a portion at near the top that is substantially flat which facilitates attachment of a suitable barrier.

The opening 19 may have a relatively small diameter, sufficient for gas exchange through the gas-permeable barrier 20. Without limitation, the opening 19 can be 2.5 to 15 mm in diameter. Specifically, the opening 19 may be between 5 and 10 mm in diameter.

Further, the opening 19 may particularly be located where it is not exposed to the foam created, for example at a top 18 or high point of container 10. The opening 19 may also include a microbial barrier to reduce potential exposure of the foam to airborne particles and microbes upon equilibration of pressures and withdrawal of the foam. The microbial barrier and gas permeable may be two separate elements or may be combined in a single barrier element.

In the present example, the barrier is not manipulated during normal use. The barrier 21 may be manipulated. In order to close off the female coupling member with lid 16, the barrier 21 may either be pierced or removed.

In other examples, instead of a gas-permeable barrier, a configuration such as the one illustrated in WO2020038928 is provided, i.e., a pressure release may be provided in the form of an orifice with a releasable cover. Such an orifice may have a relatively small diameter, sufficient for gas exchange. Without limitation, orifice can be 0.5 to 5 mm in diameter. Specifically, orifice 19 may be between 1 and 3 mm in diameter. The releasable cover, may be an aluminium foil sealed over the orifice.

In yet other examples, the pressure release may be a relief valve, or an orifice with a breakable or removable cover or tab. The function of the pressure release is to balance a pressure inside container 10 and a pressure outside the container. Equilibration of pressures can avoid potential formation of undesirable large bubbles in the sclerosant foam.

The mixing component 30 configured for rotating on the bottom 11 of the container may comprise a magnetic element, in particular a magnetic core. The magnetic element may be provided in a receptacle, e.g. a centrally arranged receptacle. The magnetic element may be a permanent magnet or may be made from a (soft) ferromagnetic material. When using a (soft) ferromagnetic material, rather than a permanent magnet, the magnetic element obtains its magnetic properties because of its arrangement in the presence of a magnet, e.g. the magnet of a magnetic stirrer. The magnetic properties then disappear when the magnet is removed. The magnetic element may be made e.g. from neodymium, or other materials with good magnetic properties. In another example, the magnetic element may be made of stainless steel.

The mixing component 30 may comprise a plurality of suitably shaped features for mixing. Such features may include e.g. blades, rings or teeth, which ensure mixing a liquid and gas by creating sufficient turbulence and mixing of the various components as the component is rotated. Mixing may be increased by forced passage through openings or spaces in the disc.

The mixing component may in some examples be freely arranged, i.e. not supported or suspended by any element on the sidewall or the top of the container. The mixing component 30 may comprise a central protrusion which is configured to reduce contact and friction between a bottom of the mixing element with the bottom of the container. The protrusion may be tapered and pointed. When the mixing element starts to rotate, the protrusion maintains contact with the bottom of the container body.

In examples, the mixing component 30 may be a disc or a disc-like component with blades, rings or teeth (or other features shaped for mixing) around an outer perimeter of the disc.

Figures 2A - 2F schematically illustrate an example of a mixing component that may be used in examples of the present disclosure. It should be clear that in other examples, other mixing components may be used. The shape of the mixing component preferably promotes effective mixing of liquid with gas.

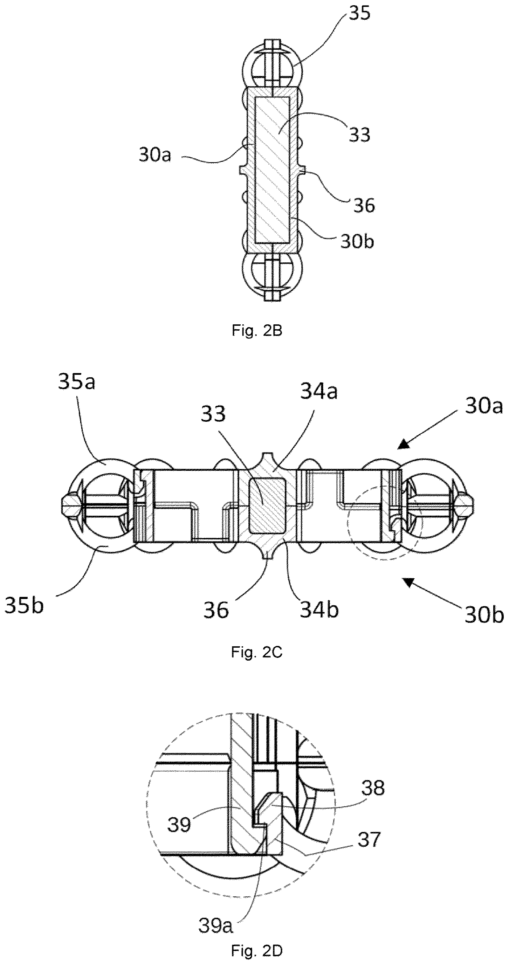

The mixing element 30 in this example may be a disc and, as may be seen e.g. in figure 2B, it may comprise a magnetic element 33, in particular a magnetic core. As shown e.g. in figure 2A, the magnetic element may be provided in a receptacle 34, e.g. a centrally arranged receptacle 34. The magnetic element 33 may be a permanent magnet or may be made from a (soft) ferromagnetic material as mentioned before.

Around an outer perimeter of the disc a plurality of suitably shaped features for mixing may be provided. Such features may include e.g. blades, rings or teeth, which ensure mixing a liquid and gas by creating sufficient turbulence and mixing of the various components as the disc is rotated. Mixing may be increased by forced passage through openings or spaces in the disc.

The disc may be freely arranged, i.e. not supported or suspended by any element on the sidewall or the top of the container. The mixing element 30 may comprise a central protrusion 36 which is configured to reduce contact and friction between a bottom of the mixing element with the bottom of the container. The protrusion 36 may be tapered and pointed. When the mixing element starts to rotate, the protrusion 36 maintains contact with the bottom of the container body.

In this particular example, ring shaped mixing features 35 are shown, but it should be clear that many other shapes may be suitable. In this particular example, as shown e.g. in figure 2C, the disc may be made by assembly of an upper half 30A and lower half 30B. Each of the halves 30A, 30B may include half rings 35A, 35B, half compartments 34A, 34B and suitable coupling features. Half compartment 34A, once coupled with half compartment 34B forms a receptacle for the magnetic element 33.

Each of the halves may be made from a suitable polymer, such as polyethylene and may be made e.g. by injection molding. As may be seen e.g. in figure 2d, the halves may include suitable joining features, e.g. fingers 37 with bulging tips 38 which can snap fit over corresponding bulges 39A on a sidewall 39 of the other half.

Figure 3 schematically illustrates a product 100 to produce a foamed sclerosant composition comprising a container as previously discussed.

The product 100 further comprises a sealed package 110 defining an interior space 112 for housing the container 10. The package 110 may include a bottom, and a plurality of sidewalls. A peelable lid 120 may be heat sealed at the top of the package to close off the interior space 112 of the package 110.

The interior space 112 is filled with a gas. Further, the interior space 112 of the sealed package 110 and the foaming space of the container 10 are in fluid communication.

As previously discussed regarding the container 10, the product 100 may be sterilized following any suitable procedure known in the art.

In some examples, the gas in the interior space 112 has a composition which substantially corresponds to the composition in the foaming space of container 10. In specific examples, the gas in the interior space 112 may comprise a percentage of O₂ between 70 % - 50 % (more specifically 65%), and a percentage of CO₂ between 30 % - 50 % (more specifically 35%). Since the gas inside the foaming space of the container 10 and in the interior space 112 of the sealed sterile package 110 may have substantially the same composition, the mass transfer between these two volumes of gas through the gas-permeable barrier does not change the gas composition inside the container 10 in which foaming will occur.

As illustrated in figure 3 with a broken line, the sealed package may comprise a lid 120 configured to be peeled off by a user of the product, e.g. a medical professional. Once the package 110 is open to the atmosphere, the mass transfer across the gas-permeable barrier 20 of the container 10 will, after some time, result in a change in the composition of the gases inside the container 10. Thus, it may be preferable to use the container 10 to form a foam during the first 15 min after opening the package 110, e.g. peeling off the lid 120, and more specifically during the first 5 min after opening the package 110.

A suitable time may be determined depending on the barrier. In some examples, a suitable time will be chosen such that the percentage of atmospheric air in the foaming space of the container when preparing the foam is less than 10 %, specifically less than 5 % and preferably less than 2 % or less than 1%.

The sealed package 110 may comprise a gas-impermeable layer, or otherwise be made impermeable to gas. Thus, the sterile package 110 may remain with substantially the same gas composition during a prolonged period of time, e.g. months or even years. In the present example, the gas-impermeable layer may be part of a multi-layer arrangement, further providing rigidity to the package 110 and protecting the container 10, e.g. against sun radiation. More precisely, the illustrated example comprises a multi-layer arrangement comprising an exterior layer of polyethylene, an intermediate layer of ethylene-vinyl alcohol copolymer, and an interior layer of polyethylene. Other examples may comprise different arrangements of the layer(s), such as a single gas-impermeable layer.

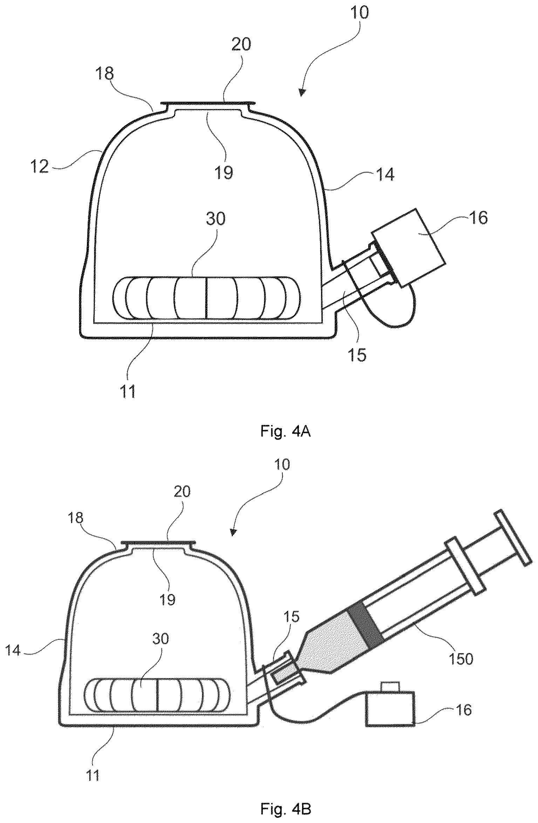

An example of a method for preparing a sclerosant foam with a product 100 according to the present disclosure is illustrated in figures 4A - 4F. Thus, figures 4A to 4F illustrate a set of chronological steps to prepare a sclerosant foam according to the present disclosure. Note that intermediate steps may be included into the foam preparation. Further note that the foam has been schematically illustrated as an area with a pattern of empty circles and that atmospheric air into the container 10 has been illustrated as an area with a pattern of dots.

In figure 4A, a product 100 according to the previously shown examples or similar is provided. The product 100 is filled with a mixture of physiological gases and comprises a container 10 in fluid communication with the interior space of the product 100 through a gas-permeable barrier 20. In the illustrated example, the container 10 comprises a stopper 16 to protect the female coupling member 15 against the ingress of contaminants. Once the package 110 is opened and the container 10 is extracted, the stopper 16 may be removed to prepare the female coupling member 15 for the introduction of a tip of a syringe.

At this point, the container 10 will be in fluid communication with the atmospheric air at least through the gas-permeable barrier 20. Further, the container 10 may also be in fluid communication with the atmospheric air through the female coupling member 15, depending on the characteristics of the same. Thus, to limit the diffusion of the mixture of physiological gases from the container 10 into the atmosphere, it is preferable to continue with the steps of the foam preparation in a relatively short period of time, e.g. less than 10 min or less than 15 minutes. Depending on the barrier used, and depending on the cross-sectional area of the opening(s), the time that may pass may be shorter or longer than 10 or 15 minutes.

The time that may be allowed to pass may also depend on the desirable composition of the foamed sclerosant composition. For some treatments, a composition of gas inside container during foaming should be substantially 100%, i.e. 100% or very close to it. The composition of the bubbles in the foam will have a mixture of gases that is substantially the same as the composition of the gas inside the container. Foaming should thus occur immediately or almost immediately after opening the package and/or removing the container from the package. For other treatments, a mixture of physiological gases and e.g. 2%, 5%, or a higher percentage of air may be acceptable.

Figure 4B illustrates how a syringe 150 is connected to the female coupling member 15. Thus, the method for preparing a sclerosant foam may also comprise providing a syringe 150 filled with a sclerosant liquid composition. In an example, such a syringe 150 may be provided as a pre-filled syringe, possibly as part of the product 100. Alternatively, the syringe 150 may be filled with the sclerosant liquid immediately prior to the preparation of the foam.

In one particular example, the sclerosing composition may comprise a solution of a sclerosing agent, such as e.g. polidocanol, in a liquid, such as water or physiological saline, at a concentration from 2 mg to 30 mg in 1 mL liquid (which corresponds to 0.20 - 3.0 %) (w/v). In another example, the sclerosing composition may comprise a solution of a sclerosing agent, such as polidocanol, in a liquid, such as water or physiological saline, at a concentration from 2 mg to 5 mg in 1 mL liquid (which corresponds to 0.20 - 0.50 % (w/v)). With the devices and methods described herein, it has been found that even at very low concentrations e.g. 0.2 % or 0.3 % (w/v), still a stable foam may be obtained, contrary to e.g. Tessari's method.

In another particular example, the sclerosing composition may comprise a solution of polidocanol in water or physiological saline at a concentration of 5 mg/mL.

In another particular example, the sclerosing composition may comprise a solution of polidocanol in water or physiological saline at a concentration of 20 mg/mL.

In another particular example, the liquid composition may include Sodium tetradecyl sulfate (STS) at a concentration of 0.2 - 3 %.

The volume of the syringe may be e.g. 5 cc, 10 cc or 20 cc. The syringe may comprise e.g. 2 ml or more of liquid sclerosant composition.

As discussed, the syringe 150 may be mated with the female coupling member 15 by insertion of the syringe 150. In further examples, the container body may include a one-way valve or a selectively displaceable element to avoid any contamination.

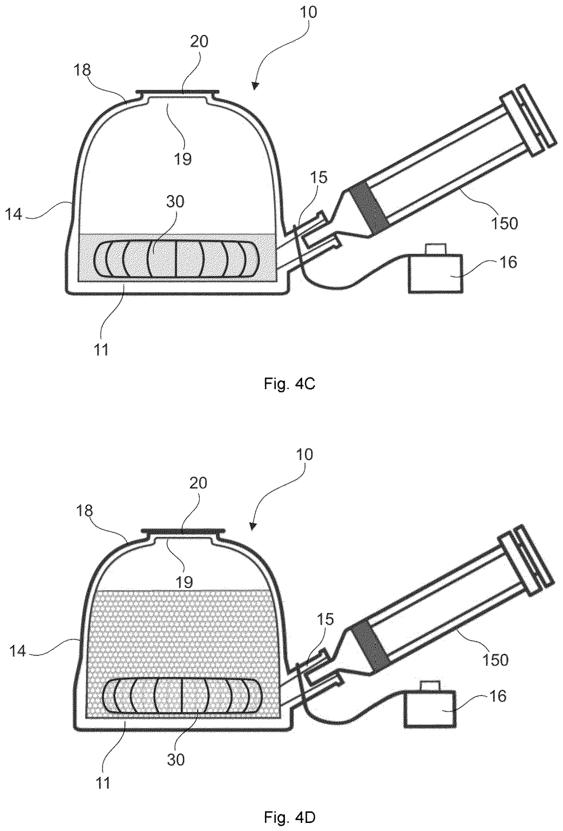

As illustrated in figure 4C, once a leak-free fitting between the syringe 150 and the female coupling member 15 is established, the liquid composition inside the syringe 150 may be injected and can enter the foaming space. Depending on the rate of liquid injection, a slight overpressure may be created inside the foaming space, that is, the pressure inside the foaming space may be higher than the external atmospheric pressure. However, this slight overpressure will be quickly equalized due to the presence of the gas-permeable barrier 20. Further, any air or gas inside the syringe 150 may also be injected into the foaming space, although this may be done at any moment prior to the extraction of the foam.

The previously described steps may be carried out in particular after the placement of the container 10 on a magnetic stirrer. This may be a standard magnetic stirrer which may typically be found in many laboratories. In some other examples, the magnetic stirrer may be specifically adapted to the container and may include a receptacle for receiving the container 10. A slight press fit between the container 10 and the receptacle may be provided. The magnetic stirrer may be a stirrer with a magnet driven by a motor, but may also be a magnetic stirrer which is based on induction using magnetic coils which provide for a rotating magnetic field.

As illustrated in figure 4D, the magnetic stirrer may then be activated. The mixing component 30 may thus be set in rotation. The mixing component 30, e.g. the disc with magnetic element that was previously described, may rotate on the bottom 11 of the container 10. Depending on the type of foam to be created, depending on the liquid sclerosant composition, and depending on the gas in the foaming space, different revolution speeds and times may be used. In some examples, the magnetic stirrer may include a memory with a number of predefined programs suitable for different foams, such that a user only needs to select a suitable program.

It is noted that it can be advantageous not to remove the syringe 150 during the formation of the foam and maintain it coupled to the container 10.

In particular examples, the magnetic stirrer is first set to a high speed for foam creation, which is reached in a short time, for example a few seconds. Such a high speed may be between 1,500 and 5,000 RPM, and particularly between 2,000 and 4,000 RPM. After as little as 15 seconds, foam may be created.

In order to increase the stability and quality of the foam, the high speed of rotation may be maintained, however. And as will be explained herein, the high speed of rotation may also be varied. After suitable creation of foam, the speed of the magnetic stirrer may be reduced to a speed which may be chosen such that the foam that has been created is pushed to the outer perimeter of the foaming space due to centrifugal forces. The second speed may be between 200 - 2000 RPM in examples. In an example, this reduced speed may be lower than 1,500 RPM. In an example, the speed may be between 200 and 1,200 RPM, specifically around 1,000 RPM.

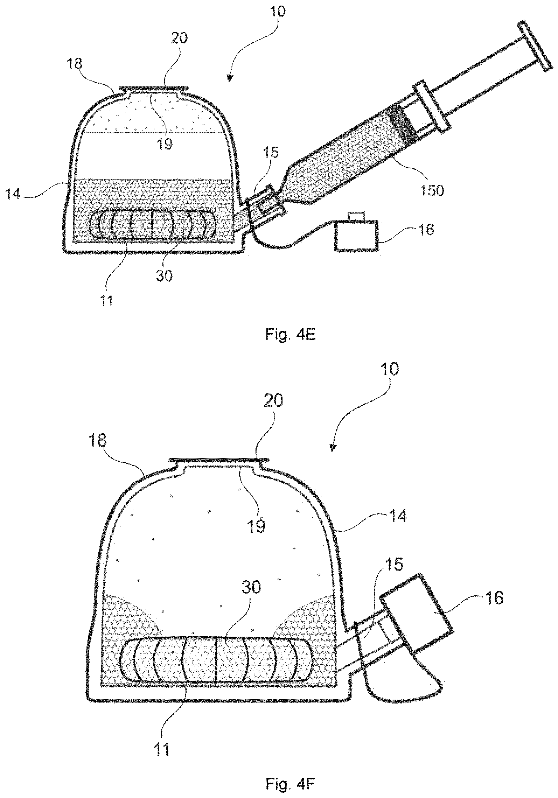

As illustrated in figure 4E, once the foam has been stabilized, the foam may be extracted from the container 10. Note that the magnetic stirrer may operate while the foam is extracted. As the gas-permeable barrier 20 allows pressure equalization between an inside of the container 10 and atmospheric pressure, the potential formation of undesirable large bubbles in the sclerosant foam within the collection syringe is avoided.

After extraction of the sclerosant foam, the foam may be used in the treatment of a patient.

The volume of container 10 as disclosed herein may be, for example, 30 ml. Such a container was used in various experiments. With methods according to the examples disclosed herein, approximately 10 ml of stable foam was obtained and easily extracted when introducing 2 ml of liquid sclerosant composition (of any of the hereinbefore mentioned concentrations).

When introducing 4 ml of liquid sclerosant composition, about 20 ml of stable foam was obtained and easily extracted. As illustrated in figure 4F, the foam may be extracted in a single step or in multiple steps. For example, a first volume of foam may be extracted and may be used as a first dose in the treatment of a patient. Then, the remaining foam me be kept in the container 10 while the mixing component 30 keeps rotating. Thus, the foam may remain stable for a later use, e.g. a second dose in the treatment of a patient. The stopper 16 may be coupled to the female coupling member 15 to avoid foam contamination and foam spilling between subsequent foam extractions.

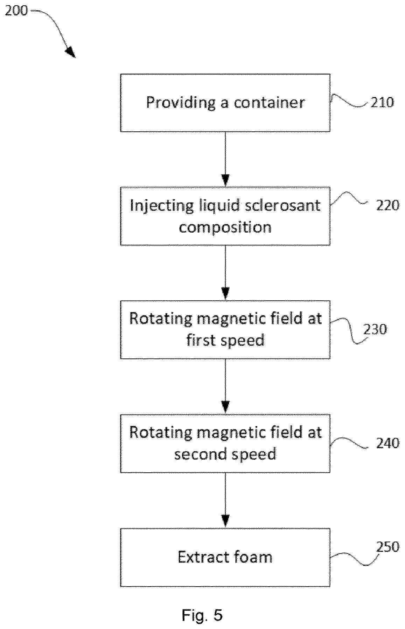

Figure 5 schematically illustrates an example of a method 200 for preparing a foamed sclerosant composition. The method 200 for preparing a foamed sclerosant composition comprises, at block 210, providing a container having one or more sidewalls extending between a top and a bottom of the container body, with a foaming space being defined in an interior of the container body, and wherein the foaming space contains a mixing component configured for rotating on the bottom of the container body and comprising a magnetic part and a shape suitable for mixing. The container may be a container such as illustrated e.g. in figures 1 - 4.

The method further comprises, at block 220, injecting a liquid sclerosant composition into the foaming space of the container. Injecting the liquid may be carried out as previously illustrated with respect to figures 1, 3 and 4.

The method 200 further comprises providing a rotating magnetic field in order to rotate the mixing component in the foaming space. Providing the rotating magnetic field comprises, at block 230, providing a rotating magnetic field at a first speed, and maintaining the rotating magnetic field at the first speed for a first holding time. The method 200 further comprises, at block 240, providing a rotating magnetic field at a second speed, the second speed being higher at a first speed, and maintaining the rotating magnetic field at the second speed for a second holding time.

In some examples, the method 200 may further comprise, at block 250, extracting the foam from the container.

In examples, as delivered, the container may comprise air. In other examples, as delivered, the container may comprise a sterile physiological gas or mixture of physiological gases, and optionally is a mixture of O₂, and CO₂. In examples, a percentage of O₂ may be between 70 % - 50 % and a percentage of CO₂ may be between 30 % - 50 %. In some examples, the percentage of O₂ may be approximately 65% and the percentage of CO₂ may be approximately 35%.

In some examples, the first holding time may be at least 10 seconds, and particularly between 10 and 40 seconds, and more particularly the first holding time may be at least 15 seconds, and specifically between 15 and 30 seconds. A holding time may herein be understood as a time during which a speed of rotation is substantially constant. A constant speed of rotation may be understood as a variation in speed of less than 15%. For example, a first speed of rotation may be 2.000 RPM. During the holding time, the speed of rotation may vary a little but may not be lower than 1.700 RPM and not be higher than 2.300 RPM.

In some examples, the first speed may be above 1.500 RPM, and particularly the first speed may be between 1.500 RPM and 2.500 RPM. Depending on the concentration of the sclerosant liquid, the minimum speed for generating a foam may vary. Particularly, for polidocanol concentrations below 1%, a minimum speed required for the creation of foam may be around 1.500 RPM. For higher concentrations of polidocanol, foam may be obtained at lower speeds, but at relatively low liquid:gas ratios. For example, for polidocanol at a concentration of 3%, a minimum speed required for the creation of foam may be around 1.000 RPM. In general, for concentrations of polidocanol below 3%, a suitable speed may be 2.000 RPM or higher. For sodium tetradecyl sulfate at different concentrations, a suitable speed may be 1.500 or higher.

After a certain stabilization with the first holding time, the speed of rotation of the magnetic field may be increased to increase the liquid:gas ratio in the obtained foam. In examples, the second speed may be above 3.000 RPM, and particularly wherein the second speed may be between 3.000 RPM and 4.200 RPM. For example, for a polidocanol concentration of 1% at a rotation of 2.000 RPM, a foam with a liquid:gas ratio obtained may be about 1:4 and at a rotation of 2.500 RPM, a ratio of 1:4,5 may be found. With a further increase in speed, e.g. 3.000 RPM a ratio of 1:5 may be found, and a maximum that has been found in a "single stage" production of foam is about 1:5,5.

However, by adding a staged increase of speed, after a first speed of 2.000 RPM, and a second speed of 3.000, 3.500 or 4.000 RPM, the obtained ratio of liquid:gas may be increased to between 1:6,5 - 1:8,5.

Without wishing to be bound to any theory, one theory for the fact that the staged increased in speed increases the liquid:gas ratio is the following: bubbles in the sclerosant foam are composed of a layer or skin of molecules arranged so that the hydrophobic end faces the gas and the hydrophilic end faces the liquid interphase. In this liquid interphase, the sclerosant molecules are organized in the form of micelles and/or free molecules mixed with water molecules or other components. A micelle maintains a circular spherical structure where its hydrophobic portion is oriented towards the center and the hydrophobic portion towards the outside.

When the sclerosant becomes a foam, there is an equilibrium between the bubbles (formed by liquid and gas molecules) and the liquid molecules exclusively between the bubbles themselves. It is important to note that when making foam, many sclerosant molecules remain in liquid form and, therefore, are potentially convertible again into new bubbles. When the foam has formed under certain conditions and is maintained at a stable speed, there are sclerosant molecules that have remained in a liquid state between bubbles. If in this situation the agitation speed is increased and therefore the shearing of these molecules that are in a liquid state is increased, new bubbles are formed since this energy favors their reorganization.

The second holding time may be at least 15 seconds, and particularly may be at least 20 seconds, or more particularly at least 25 seconds. In order to create further bubbles (and thereby increase the liquid:gas ratio), a minimum period of time is necessary. After e.g. about 20 seconds or 30 seconds, a maximum may be reached.

In some examples, the liquid sclerosant composition may comprise polidocanol. Although STS may be used, with the staged increase of rotation, the liquid:gas ratio may become very high and the obtained foam (depending on the intended use) may be too dry.

In one particular example, the sclerosing composition may comprise a solution of a sclerosing agent, such as e.g. polidocanol, in a liquid, such as water or physiological saline, at a concentration from 2 mg to 30 mg in 1 mL liquid (which corresponds to 0.20 - 3.0 %) (w/v). In another example, the sclerosing composition may comprise a solution of a sclerosing agent, such as polidocanol, in a liquid, such as water or physiological saline, at a concentration from 2 mg to 5 mg in 1 mL liquid (which corresponds to 0.20 - 0.50 % (w/v)). With the devices and methods described herein, it has been found that even at very low concentrations e.g. 0.2 % or 0.3 % (w/v), still a stable foam may be obtained, contrary to e.g. Tessari's method.

The volume of the syringe for injection of the liquid sclerosant composition may be e.g. 5 cc, 10 cc or 20 cc. The syringe may comprise e.g. 2 ml or 2,5 ml of liquid sclerosant composition.

In some examples, the method may further comprise extracting the foam, and particularly wherein the foam is extracted in presence of a rotating magnetic field. The foam may be extracted while the magnetic field is rotating at a third speed, wherein the third speed is lower than the second speed. In specific examples, the third speed may be the same or substantially the same as the first speed. In other examples, the third speed may be lower than the first speed.

In a further aspect, a magnetic stirrer is provided which comprises a support for placement of a container with a foaming space being defined in an interior of the container body, and wherein the foaming space contains a mixing component. The magnetic stirrer further comprises an actuator for providing a magnetic field and a control system.

The support for the placement of the container may be a simple flat plate. In other examples, the support may comprise a grip or recess in which the container may be fitted.

The rotating magnetic field may be created by actuation of a motor which actively drives rotation of a permanent magnet. In other examples, the rotating magnetic field may be created through a plurality of coils, and by varying electric current through the coils.

The control of the magnetic stirrer may include a processor and memory wherein the memory includes instructions corresponding to one or more predetermined rotation programs, such that when the instructions are executed by the processor, the magnets create a rotating magnetic field according to the predetermined rotation programs.

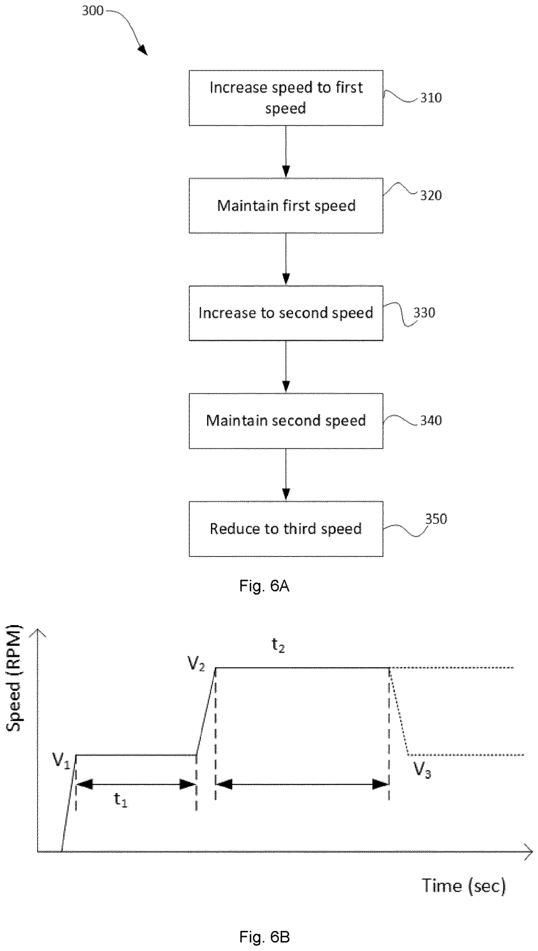

The predetermined rotation programs may correspond to the sequences described herein and may be further illustrated with reference to figures 6A and 6B.

The magnetic stirrer may further comprise a user interface, wherein the user interface enables selection by a user of the predetermined rotation programs. The user interface may include a screen display, and particularly a touch screen display.

The predetermined rotation programs may be identified by one or more of the following: the gas in the foaming space, and the liquid sclerosant composition, specifically a concentration of the drug in the liquid sclerosant composition.

The control system is configured to control the actuator such that a rotating magnetic field is provided which is configured to rotate the mixing component in the foaming space; wherein the rotating magnetic field comprises: a rotating magnetic field at a first speed, during a first holding time; and a rotating magnetic field at a second speed during a second holding time, wherein the second speed is higher at a first speed, and maintaining the rotating magnetic field at the second speed for a second holding time.

As illustrated in figure 6A, the speed of rotation may be increased to a first speed, V₁ at block 310. This increase may be relatively rapid, e.g. within a few seconds. Any magnetic stirrer will have an inevitable delay which may be longer for a mechanically driven stirrer than for a magnetic stirrer based on induction. This is illustrated with the relatively steep increase to V₁. It should be borne in mind that figure 6B is not drawn to scale necessarily.

At block 320, the first speed may be maintained for a first holding time t₁. The first speed may be above 1.500 RPM, and particularly wherein the first speed is between 1.500 RPM and 3.000 RPM, more particularly between 1.500 - 2.500 RPM. The first holding time t₁ may be e.g., between 10 and 40 seconds, particularly between 15 and 30 seconds. There is no specific upper limit to the first holding time. In general there is no effect (or only a very minor effect) on the liquid:gas ratio if the holding time is increased beyond e.g. 40 seconds or a minute.

At block 330, the speed of rotation may be increased (relatively rapidly) to a second speed of rotation V₂. Also in this case, there will be an inevitable (small) delay before reaching V₂. Although it is generally preferred to increase the speed as quickly as possible, it is also possible to more gradually increase the speed. The effect on the resulting foam however has not been significant.

The second speed is higher than the first speed and may be above 3.000 RPM, and particularly wherein the second speed is between 3.000 RPM and 4.200 RPM. The second speed may be maintained, at block 340. After the second holding time t₂, the foam may have stabilized and may be extracted. The second holding time may be at least 20 seconds, and in specific examples may be about 30 seconds. There is no specific upper limit to the second holding time, but foam production may render good results and be efficient with a range of e.g. 20 - 40, 20 - 50 or 20 - 60 seconds. After the second holding time, at block 350, the speed of rotation may be reduced to a third speed V₃.

In the example illustrated in figure 6B, the third speed V₃ has been selected to be substantially equal to the first speed V₁. The third speed may also be chosen slightly higher, and may particularly also be chosen to be lower. The third speed may be selected high enough so that the foam is forced towards the sidewall(s) of the container for ease of extraction.

Note that the technical features disclosed in relation to the containers, products, kits, systems and methods may be interchangeably used. For example, a technical feature disclosed in relation to a container may be integrated into a method to manufacture a product and *vice versa.*

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A method for preparing a foamed sclerosant composition, comprising:
providing a container having one or more sidewalls extending between a top and a bottom of the container body, with a foaming space being defined in an interior of the container body, and wherein the foaming space contains a mixing component configured for rotating on the bottom of the container body and comprising a magnetic part and a shape suitable for mixing;
introducing a liquid sclerosant composition into the foaming space of the container; and
providing a rotating magnetic field in order to rotate the mixing component in the foaming space; wherein
providing the rotating magnetic field comprises
providing a rotating magnetic field at a first speed,
maintaining the rotating magnetic field at the first speed for a first holding time;
providing a rotating magnetic field at a second speed, the second speed being higher than a first speed, and maintaining the rotating magnetic field at the second speed for a second holding time.

2. The method according to claim 1, wherein the liquid sclerosant composition comprises polidocanol.

3. The method according to claim 2, wherein the concentration of polidocanol is 1%.

4. The method according to any of claims 1 - 3, wherein the container comprises a sterile physiological gas or mixture of sterile physiological gases, and optionally is a mixture of O₂, and CO₂.

5. The container according to claim 4, wherein a percentage of O₂ is between 70 % - 50 % and a percentage of CO₂ is between 30 % - 50 %, and particularly wherein the percentage of O₂ is approximately 65% and the percentage of CO₂ is approximately 35%, and particularly with less than 0.8% of N₂.

6. The method according to any of claims 1 - 5, wherein the first speed is above 1.500 RPM, specifically between 1.500 RPM and 4.200 RPM, and particularly wherein the first speed is between 1.500 RPM and 2.500 RPM.

7. The method according to any of claims 1 - 6, wherein the second speed is above 3.000 RPM, and particularly wherein the second speed is between 3.000 RPM and 4.200 RPM.

8. The method according to any of claims 1 - 7, wherein the first holding time is at least 10 seconds, and particularly is between 10 and 40 seconds, and more particularly the first holding time is between 15 and 30 seconds.

9. The method according to any of claims 1 - 8, wherein the second holding time is at least 15 seconds, and particularly is at least 20 seconds, more particularly at least 25 seconds.

10. The method according to any of claims 1 - 9, further comprising extracting the foam, and particularly wherein the foam is extracted in presence of a rotating magnetic field.

11. The method according to claim 10, wherein the foam is extracted while the magnetic field is rotating at a third speed, wherein the third speed is lower than the second speed.

12. A foamed sclerosant composition obtainable by a method according to any of claims 1 - 11, wherein the liquid:gas ratio in the foamed sclerosant composition is at least 1:6, particularly 1:7 or more.

13. A magnetic stirrer comprising:
a support for placement of a container with a foaming space being defined in an interior of the container, and wherein the foaming space contains a mixing component;
an actuator for providing a magnetic field; and
a control system configured to control the actuator such that a rotating magnetic field is provided which is configured to rotate the mixing component in the foaming space; wherein the rotating magnetic field comprises:
a rotating magnetic field at a first speed, during a first holding time; and
a rotating magnetic field at a second speed during a second holding time, wherein the second speed is higher at a first speed, and maintaining the rotating magnetic field at the second speed for a second holding time.

14. The magnetic stirrer according to claim 13, wherein
the first speed is above 1.500 RPM, and particularly wherein the first speed is between 1.500 RPM and 2.500 RPM, and wherein
the second speed is above 3.000 RPM, and particularly wherein the second speed is between 3.000 RPM and 4.200 RPM.

15. The magnetic stirrer according to claim 14, wherein
the first holding time is at least 10 seconds, and particularly is between 10 and 40 seconds, and more particularly the first holding time is between 15 and 30 seconds and wherein
the second holding time is at least 10 seconds, and particularly is at least 15 seconds, more particularly at least 20 seconds.
